# EUROPEAN PATENT SPECIFICATION

(11) **EP 4 285 962 B1**
(45) Date of publication and mention of the grant of the patent: **10.12.2025**
(21) Application number: 21923422.6
(22) Date of filing: 10.12.2021
(51) Int. Cl.: A61M 5/30, A61M 5/20, A61M 39/24, A61M 5/315

(54) **NEEDLE-FREE INJECTOR BASED ON UNDERWATER DISCHARGE USING SHOCK WAVE**
NADELLOSER INJEKTOR AUF BASIS VON UNTERWASSERENTLADUNG MIT STOSSWELLEN
INJECTEUR SANS AIGUILLE BASÉ SUR UNE DÉCHARGE SOUS L'EAU À L'AIDE D'UNE ONDE DE CHOC

(30) Priority: 26.01.2021 KR 20210010546
(43) Date of publication of application: 06.12.2023
(73) Proprietor: Jeisys Medical Inc., Geumcheon-gu Seoul 08501 (KR)
(72) Inventor: SEO, Kyu Young, Seoul 08501 (KR); LEE, Seung Wook, Seoul 08501 (KR); KIM, Si Youn, Seoul 08501 (KR); KIM, Min Young, Seoul 08501 (KR); YI, Won Ju, Seoul 08501 (KR); KANG, Dong Hwan, Seoul 08501 (KR)
(74) Representative: dompatent
(86) International application number: PCT/KR2021/018815
(87) International publication number: WO 2022/164016

(56) References cited:
- EP-B1- 1 599 242
- KR-A- 20190 121 486
- KR-A- 20190 128 338
- KR-A- 20190 128 338
- KR-B1- 101 207 977
- KR-B1- 102 149 190
- KR-B1- 102 170 767
- KR-Y1- 200 379 423
- US-A1- 2004 162 517

## Description

### [TECHNICAL FIELD]

Embodiments of the inventive concept described herein relate to a needle free injector based on underwater discharge using shock wave.

### [BACKGROUND ART]

A drug delivery system is a system to effectively deliver a required amount of medicine into a body by minimizing a side effect made in a conventional manner and maximizing a treatment effect by medicine, when the medicine is used to treat human diseases or wounds.

In the medicine delivery system, is the injection manner is most commonly used as it can accurately and effectively administer drugs. However, the injection manner has several problems: fear of injection due to pain during injection, risk of infection due to reuse, and the generation of a large amount of medical waste.

To solve the problems, a medicine delivering manner, such as a needle free injector, has been developed.

For example, a driving fluid injecting technology, which is one of a needle free injection technologies (NFIT), is to apply shock wave through lasers or electric waves into a driving fluid such that the driving fluid is thermally expanded to generate a strand of high-speed driving fluid using pressure, thereby injecting the driving fluid into the skin.

However, it is difficult to accurately control the density of the driving fluid, the thermal conductivity, that is, the expansion degree of the driving fluid, depending on a temperature type.

When a laser pulse having higher energy and a shorter pulse width is used to generate shock wave in the driving fluid, laser equipment is necessary. Accordingly, the size of the equipment may be increased, and the equipment price may be increased. To irradiate laser beam into the driving fluid, a larger number of optical systems are required, thereby causing the damage to the optical systems.

A similar systems is known from KR20190128338A relating to a micro-jet injection device for injecting an injection liquid stored therein in the form of high-speed micro-jet. More specifically, the micro-jet injection device applies electrical energy to a working fluid in a closed chamber to generate bubbles and uses the bubbles as the driving force to micro-jet the liquid. Dielectric breakdown occurs more easily by pre-generating microbubbles around an electrode prior to application of a high voltage for dielectric breakdown. The micro-jet injection device provided in the present invention comprises: a pressure chamber in which a working fluid is tightly filled in a sealed inner space; a drug chamber which is disposed adjacent to the pressure chamber and has a drug solution accommodated therein and a micro nozzle discharging the drug solution to the outside at one side; an elastic membrane which separates and partitions between the pressure chamber and the drug chamber as a membrane having an elasticity to allow expansion and restoration; an electrode which applies electrical energy to the working fluid in the pressure chamber to generate bubbles due to dielectric breakdown in the working fluid; and a microbubble generator which is installed inside the pressure chamber to generate bubbles of a fine size in the working fluid around the electrode.

A similar systems is known from EP1599242B1, wherein the invention relates to a device for carrying out the needleless injection of a dust-like or powdery substance into a body tissue.

A similar system is known from KR102170767B1 wherein a needleless injection device for injecting a drug, comprising: a cylindrical injection device main body having a first space and a second space along a longitudinal direction is known.

.A similar system is known from US20040162517A1, disclosing a device for performing a needleless hypodermic injection of a liquid medication contained in the device. The device includes pyrotechnical means for generating within the device a pressure necessary for injecting the medication.

### [DETAILED DESCRIPTION OF THE INVENTION]

### [TECHINICAL PROBLEM]

Embodiments of the inventive concept provide a needle free injector based on underwater discharge using shock wave, which is realized as smaller-sized and economical equipment, and prevents an optical system from being damaged.

The objects of the inventive concept are not limited to the above, but other effects, which are not mentioned, will be apparently understood to those skilled in the art.

### [TECHNICAL SOLUTION]

According to an embodiment of the inventive concept, a needle free injector based on underwater discharge and using shock wave, may include a pressure chamber in which a driving fluid is stored, a medicine chamber communicating with the pressure chamber to store medicine, a pressure transmitting unit interposed between the pressure chamber and the medicine chamber to apply propulsion pressure to the medicine stored in the medicine chamber, when a volume of the pressure chamber is expanded, an injecting unit provided at one side of the medicine chamber to discharge the medicine, and an electrode unit to expand the volume of the pressure chamber, by generating bubbles in the driving fluid stored in the pressure chamber and causing break down. The present invention is defined in claim 1

In addition, the electrode unit may include a first electrode and a second electrode to generate the shock wave, and an insulating unit interposed between the first electrode and the second electrode.

In addition, the first electrode, the second electrode, and the insulating unit may have mutually different lengths.

Further, an end portion of the first electrode, an end portion of the second electrode, and an end portion of the insulating unit, which are disposed outside the pressure chamber, are arranged to form stepped portions among the end portions.

In addition, the shock wave may be a pulse shock wave.

In addition, the pressure transmitting unit may be an elastic plate.

Further, the first electrode may be inserted into the insulating unit, and the second electrode may be disposed on an outer surface of the insulating unit.

In addition, the needle free injector may further include a driving fluid storage unit to store a driving fluid, and a driving fluid circulating unit to circulate the driving fluid stored in the driving fluid storage unit and the driving fluid stored in the pressure chamber.

In addition, the needle free injector may further include a medicine storage unit to store the medicine provided in the medicine chamber, and a check valve to prevent the medicine, which is received in the medicine chamber, from flowing back to the medicine storage unit.

In addition, the needle free injector may further include a power unit to supply pulsed power to the electrode unit, and the power unit may include a power supply unit, an electricity storage unit to store voltage and current supplied from the power supply unit in a form of electrical energy, and a switch to apply the electrical energy, which is stored in the electricity storage unit, in a form of pulsed power.

### [ADVANTAGEOUS EFFECTS OF THE INVENTION]

According to an embodiment of the inventive concept, the needle free injector based on underwater discharge and using the shock wave may be realized using smaller-size and economical equipment, and may prevent the optical system from being broken.

The effects of the inventive concept are not limited to the above, but other effects, which are not mentioned, will be apparently understood to those skilled in the art.

### [DESCRIPTION OF THE DRAWINGS]

FIG. 1 is a schematic view illustrating a needle free injector based on underwater discharge and using shock wave, according to an embodiment of the inventive concept;
FIG. 2 is a perspective view illustrating an electrode unit, according to an embodiment of the inventive concept;
FIG. 3 is a perspective view illustrating an electrode unit, according to an embodiment of the inventive concept; and
FIGS. 4 to 5 are views illustrating the operation of a needle free injector based on underwater discharge and using shock wave, according to an embodiment of the inventive concept.

### [BEST MODE]

The above and other aspects, features and advantages of the inventive concept will become apparent from embodiments to be described in detail in conjunction with the accompanying drawings. The inventive concept, however, may be embodied in various different forms, and should not be construed as being limited only to the illustrated embodiments. Rather, these embodiments are provided as examples so that the inventive concept will be thorough and complete, and will fully convey the scope of the inventive concept to those skilled in the art. The inventive concept may be defined by the scope of the claims.

The terms used herein are provided to describe embodiments, not intended to limit the inventive concept. In the specification, the singular forms include plural forms unless particularly mentioned. The terms "comprises" and/or "comprising" used herein do not exclude the presence or addition of one or more other components, in addition to the aforementioned components. The same reference numerals denote the same components throughout the specification. As used herein, the term "and/or" includes each of the associated components and all combinations of one or more of the associated components. It will be understood that, although the terms "first", "second", etc., may be used herein to describe various components, these components should not be limited by these terms. These terms are only used to distinguish one component from another component. Thus, a first component that is discussed below could be termed a second component without departing from the technical idea of the inventive concept.

Unless otherwise defined, all terms (including technical and scientific terms) used herein have the same meaning as commonly understood by those skilled in the art to which the inventive concept pertains. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, an embodiment of the inventive concept will be described in detail with reference to accompanying drawings.

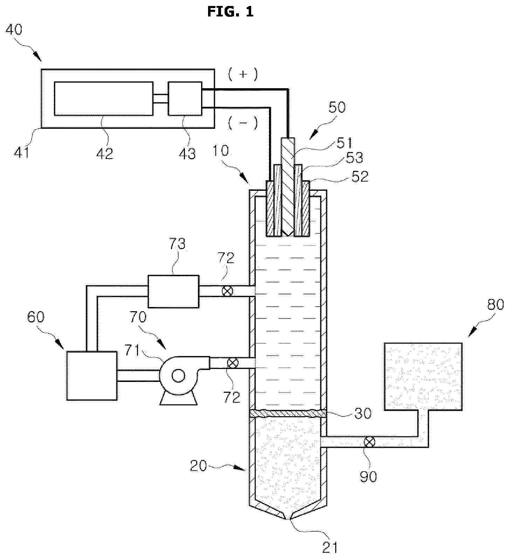

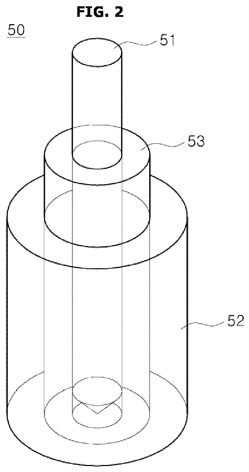

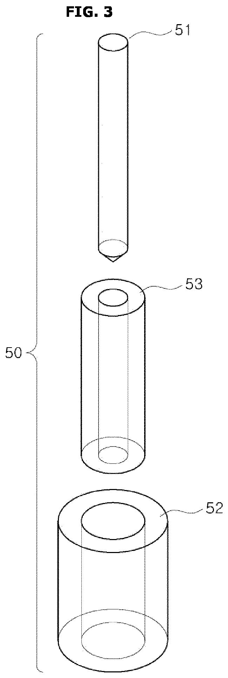

FIG. 1 is a schematic view illustrating a needle free injector based on underwater discharge and using shock wave according to an embodiment of the inventive concept. FIG. 2 is a perspective view illustrating an electrode unit, according to an embodiment of the inventive concept. FIG. 3 is a perspective view illustrating an electrode unit, according to an embodiment of the inventive concept.

As illustrated in FIG. 1, a needle free injector based on underwater discharge and using shock wave, according to an embodiment of the inventive concept includes a pressure chamber 10, a medicine chamber 20, an injecting unit 21, a pressure transmitting unit 30, a power unit 40, and an electrode unit 50.

The pressure chamber 10 and the medicine chamber 20 may be formed in a single housing, and the pressure transmitting unit 30 may be interposed between the pressure chamber 10 and the medicine chamber 20.

The pressure chamber 10 receives a driving fluid in which gas is dissolved. In detail, the pressure chamber 10 has a sealed receiving space, and the driving fluid in which gas is dissolved is received in the receiving space.

For example, the driving fluid may be water in which gas is dissolved. However, in a case in which an electrolyte or a conductive material is included in water, voltage loss of pulse shock wave may occur. Accordingly, deionized water from which electrical ions and impurities are removed may be used, or pure or ultra-pure water may be used.

For another example, the driving fluid may be alcohol, a polymer sol such as polyethylene glycol, and a liquid-phase material such as gel.

The pressure chamber 10 is not specifically limited thereof. Preferably, the pressure chamber 10 may be formed in a cylindrical shape. The electrode unit 50, which is to be described below, is inserted into one side of the pressure chamber 10, and the pressure transmitting unit 30, which is to be described below, is provided at the other side of the pressure chamber 10.

The medicine chamber 20 receives a medicine. In detail, the medicine chamber 20 has a sealed receiving space, and the driving fluid in which gas is dissolved is received in the receiving space.

The medicine chamber 20 may be formed in a cylindrical shape. The pressure transmitting unit 30, which is to be described below, is provided at one side of the medicine chamber 20, and the injecting unit 21, which is to be described below, is provided at the other side of the medicine chamber 20.

The pressure transmitting unit 30 is an elastic plate interposed between the pressure chamber 10 and the medicine chamber 20. When the volume of the pressure chamber 10 is expanded, the pressure transmitting unit 30 is stretched to apply propulsion pressure to the medicine chamber 20.

**The** pressure transmitting unit 30 may be made of a thin-film rubber material. For example, the pressure transmitting unit 30 may be formed of natural rubber or synthetic rubber that is harmless to the human body. The pressure transmitting unit 30 may be formed of a silicon material.

The injecting unit 21 is provided at one side of the medicine chamber 20 to discharge a medicine. In detail, as the volume of the pressure chamber 10 is expanded, so the pressure transmitting unit 30 is stretched, propulsion pressure is applied to the medicine stored in the medicine chamber 20. In this case, the medicine may be discharged through the injecting unit 21.

For example, the injecting unit 21 may be formed in a hole form. In addition, the injecting unit 21 protrudes, in an annular shape, from an outer portion of the medicine chamber 20. Meanwhile, although the drawing illustrates that the injecting unit 21 is provided at a lower end of the medicine chamber 20 to inject the medicine downward by way of example, the inventive concept is not limited thereto.

The injection speed of the medicine is determined depending on the diameter of the injecting unit 21. For example, the diameter of the injecting unit 21 may be in the range of 50 *µ*m to 1000 *µ*m. In this case, in a case in which the diameter of the injecting unit 21 is less than 50 *µ*m, the injecting unit 21 may inject the medicine in a smaller amount. Accordingly, the medicine may not be injected up to the sufficient depth of the skin depth part. When the diameter of the injecting unit 21 exceeds 1000 *µ*m, the injecting unit 21 may inject the medicine in a larger amount. Accordingly, as a larger amount of medicine is sprung out of the skin surface, the medicine may be wasted. Accordingly, the diameter of the injecting unit 21 is preferably limited to the above numeric value.

The power unit 40 applies voltage charged in the capacitor by the switch 43 to generate pulsed power, such that the pulsed power is transmitted to the electrode unit 50. In this case, the pulsed power refers to high-voltage electrical energy

The power unit 40 may include a power supply unit 41, an electricity storage unit 42, and the switch 43.

The power supply unit 41 may be a generator. The generator converts alternating current (AC) voltage to direct current (DC) voltage to be stored in the electricity storage unit 42.

The electricity storage unit 42 stores a voltage and a current supplied from the power supply unit 41 in the form of electricity. For example, the electricity storage unit 42 may serve as a capacitor or an inductor.

The switch 43 applies, to the electrode unit 50, pulsed power obtained by instantly (for several microseconds) boosting the electrical energy charged in the electricity storage unit 42 from lower voltage to higher voltage. In this case, the user may adjust the intensity of the voltage of the pulsed power by using the switch 43 to adjust a pulse width of pulse shock wave generated from the electrode unit 50. In this case, the user may adjust the pulse width of the pulsed power in the range from several seconds to several nanoseconds using the switch 43.

Meanwhile, the power unit 40 may further include an electric circuit to maintain the form of the generated pulse. In this case, the electric circuit may be preferably a pulse forming network (PFN) to prevent a square pulse from being deformed due to a parasitic inductance, such that the form of the pulse is maintained.

The electrode unit 50 is connected to one side of the pressure chamber 10 to receive the pulsed power from the power unit 40 and apply high-voltage current to the driving fluid, thereby generating bubbles by inducing electrolysis of the driving fluid stored in the pressure chamber 10. In addition, the electrode unit 50 causes break down in the state that the bubbles, which are generated in such a manner, is present, such that shock wave and a cavity are generated in the pressure chamber 10, thereby expanding the volume of the pressure chamber 10. The shock wave may be the pulse shock wave. In this case, the cavity may be a region including bubbles generated inside the pressure chamber 10.

In detail, when the electrode unit 50 applies the high-voltage current to the driving fluid, the bubbles are generated through the electrolysis of the driving fluid. In addition, the break down is caused in the bubbles generated in such a manner. Accordingly, as the cavity formed in the pressure chamber 10 is instantly expanded and distinguished again, the shock wave are generated to expand the volume of the pressure chamber 10. In detail, when the volume of the pressure chamber 10 is expanded, the pressure transmitting unit 30 is stretched toward the medicine chamber 20. Accordingly, as propulsion pressure is applied to the medicine stored in the medicine chamber 20, the medicine is discharged through the injecting unit 21.

Meanwhile, the power unit 40 applies high-voltage electricity to the electrode unit 50 through a control unit such as a micro-computer.

The electrode unit 50 may include a first electrode 51, a second electrode 52, and an insulating unit 53.

The first electrode 51 and the second electrode 52 are connected to one side of the pressure chamber 10 to make contact with the driving fluid and receive the pulsed power from the power unit 40. For example, the first electrode 51 and the second electrode 52 may be electrode bodies to apply "+" voltage and "-" voltage, respectively.

The insulating unit 53 is interposed between the first electrode 51 and the second electrode 52 to insulate the first electrode from the second electrode 52. For example, the insulating unit 53 may be formed in an annular shape. The first electrode 51 in the cylindrical shape is inserted into the insulating unit 53, and the second electrode 52 in the annular shape may be provided on an outer surface of the insulating unit 53. In addition, the inner surface of the insulating unit 53 may make contact with or make non-contact with the first electrode 51. Further, the outer surface of the insulating unit 53 may make contact with or make non-contact with the second electrode 52. Further, the insulating unit 53 surrounds the first electrode 51 such that the first electrode 51 is isolated from the outside. Accordingly, Joule heating is rapidly performed, such that bubbles are rapidly generated from the driving fluid.

When the pulsed power is applied to the first electrode 51 and the second electrode 52, the electrolysis occurs in the driving fluid around the first electrode 51 and the second electrode 52 to generate bubbles. Simultaneously, since the first electrode 51 is spaced apart from the second electrode 52 by the insulating unit 53, break down is caused together with spark in the bubbles generated around the first electrode 51 and the second electrode 52.

Meanwhile, one end portion of the first electrode 51, one end portion of the second electrode 52, and one end portion of the insulating unit 53, which make contact with the driving fluid, may be aligned in line with each other. Accordingly, since the one end portion of the first electrode 51 is significantly close to the one end portion of the second electrode 52, a significantly strong spark occurs between the one end portion of the first electrode 51 and the one end portion of the second electrode 52. Accordingly, the break down may be easily caused in the driving fluid between the one end portion of the first electrode 51 and the one end portion of the second electrode 52.

Meanwhile, the one end portion of the first electrode 51 is not specifically limited in shape, but may be formed in a conical shape. In this case, as the pulsed power is concentrated on the one end portion of the first electrode 51, the spark may easily occur from the bubbles generated around the first electrode 51 and the second electrode 52.

The first electrode 51, the second electrode 52, and the insulating unit 53 may have mutually different lengths.

For example, the length of the insulating unit 53 may be shorter than the length of the first electrode 51 and longer than the length of the second electrode 52.

In this case, the other end of the first electrode 51, the other end of the second electrode 52, and the other end of the insulating unit 53, which are disposed on the outside of the pressure chamber 10, are arranged to form stepped portions. Accordingly, a gap between the other end portion of the first electrode 51 and the other end portion of the second electrode 52 may be larger than the gap between the one end portion of the first electrode 51 and the one end portion of the second electrode 52. As the gaps are insulated by the insulating unit 53, the short between the other end portion of the first electrode 51 and the other end portion of the second electrode 52 may be prevented.

Meanwhile, the bubbles are generated in the driving fluid stored in the pressure chamber 10 by the pulse shock wave of the electrode unit 50. Accordingly, an amount of gas dissolved in the driving fluid may be reduced, and the internal pressure of the pressure chamber 10 may be increased. According to the present embodiment, an amount of gas dissolved in the driving fluid may be complemented and the internal pressure of the pressure chamber 10 may be reduced through a driving fluid storage unit 60 and a driving fluid circulating unit 70.

The driving fluid storage unit 60 is a storage tank to store the driving fluid having the dissolved gas.

The driving fluid circulating unit 70 circulates the driving fluid stored in the driving fluid storage unit 60 and the driving fluid stored in the pressure chamber 10. For example, the driving fluid circulating unit 70 may include a circulating pump 71, which circulates the driving fluid, which is stored in the driving fluid storage unit 60, and the driving fluid stored in the pressure chamber 10, and a pair of solenoid valves 72 provided between the driving fluid storage unit 60 and the circulating pump 71, and between the circulating pump 71 and the pressure chamber 10. In addition, the driving fluid circulating unit 70 may further include a filter 73 to filter impurities and bubbles included in the driving fluid circulating by the circulating pump 71.

According to the present embodiment, the driving fluid circulating unit 70 may further include a pressure sensor to control the circulating pump 71 and the solenoid valve 72. The pressure sensor measures internal pressure of the pressure chamber 10 while causing the spark by applying pulse shock wave to the driving fluid.

When the pressure sensor senses that the internal pressure of the pressure chamber 10 is equal to or greater than a reference value, the solenoid valve 72 may be open, the circulation of the driving fluid by the circulating pump 71 may be stopped, and the circulating pump 71 may circulate the driving fluid stored in the driving fluid storage unit 60 and the driving fluid stored in the pressure chamber 10.

Meanwhile, when the medicine stored in the medicine chamber 20 is injected through the injecting unit 21, the medicine stored in the medicine chamber 20 is necessary to be complemented. According to the present embodiment, the medicine of the medicine chamber 20 may be complemented through the medicine storage unit 80 and a check valve 90.

The medicine storage unit 80 is a storage tank having medicine stored therein.

The check valve 90 delivers the medicine stored in the medicine storage unit 80 only to the pressure chamber 10.

Hereinafter, the operation of a needle free injector based on underwater discharge using shock wave according to an embodiment of the inventive concept will be described by way of example.

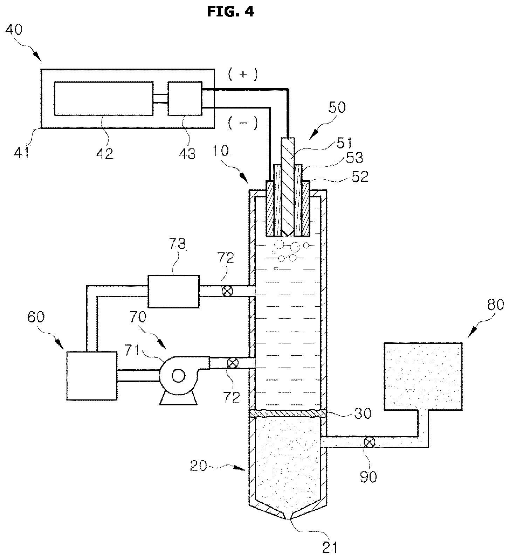

FIGS. 4 to 5 are views illustrating the operation of the needle free injector based on underwater discharge using the shock wave, according to an embodiment of the inventive concept.

As illustrated in FIG. 4, first the power unit 40 generates pulsed power and applies the pulsed power the electrode unit 50.

Next, the first electrode 51 and the second electrode 52 of the electrode unit 50 receive the pulsed power to apply high-voltage current to the driving fluid stored in the pressure chamber 10.

In this case, the electrolysis is performed with respect to the driving fluid around the first electrode 51 and the second electrode 52 to generate bubbles, break down and spark are caused in the bubbles generated in the same manner, and the cavity formed in the pressure chamber 10 is instantly expanded and distinguished again to generate pulse shock wave, such that the volume of the pressure chamber 10 is expanded.

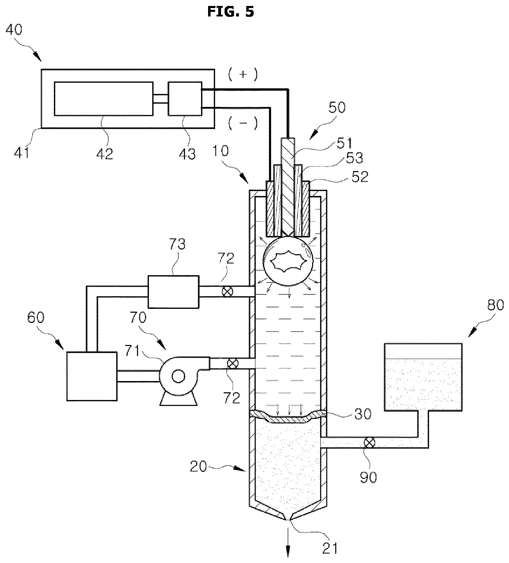

Thereafter, as illustrated in FIG. 5, as the volume of the pressure chamber 10 is expanded by the bubbles generated inside the pressure chamber 10, the pressure transmitting unit 30 is stretched, such that propulsion pressure is applied to the medicine stored in the medicine chamber 20.

Next, as the propulsion pressure is applied to the medicine stored in the medicine chamber 20, the medicine stored in the medicine chamber 20 is injected into the skin through the injecting unit 21 at an ultra-high speed.

According to the inventive concept, the needle free injector based on underwater discharge using the shock wave, according to an embodiment of the inventive concept may be realized as smaller-sized and economical equipment, and may prevent an optical system from being damaged.

Although an embodiment of the inventive concept are described with reference to the accompanying drawings, it will be understood by those skilled in the art to which the inventive concept pertains that the inventive concept can be carried out as defined in claim 1. Therefore, the present invention is defined in claim 1.

## Claims

1. A needle free injector based on underwater discharge using shock wave, the needle free injector comprising:
a pressure chamber (10) in which a driving fluid is stored;
a medicine chamber (20) communicating with the pressure chamber (10) to store medicine;
a pressure transmitting unit (30) interposed between the pressure chamber (10) and the medicine chamber (20),wherein the pressure transmitting unit (30) is applying propulsion pressure to the medicine stored in the medicine chamber (20), when a volume of the pressure chamber (10) is expanded;
an injecting unit (21) provided at one side of the medicine chamber (20) to discharge the medicine; and
an electrode unit (50) to expand the volume of the pressure chamber (10), by generating bubbles in the driving fluid stored in the pressure chamber and
causing break down,
**characterized in that** the electrode unit (50) includes:
a first electrode (51) and a second electrode (52) to generate the shock wave;
wherein the shock wave expands the volume of the pressure chamber
and
an insulating unit (53) interposed between the first electrode (51) and the second electrode (52),
wherein the first electrode (51), the second electrode (52), and the insulating unit (53) have mutually different lengths,
wherein an end portion of the first electrode (51), an end portion of the second electrode (52), and an end portion of the insulating unit (53), which are disposed outside the pressure chamber (10), are arranged to form stepped portions among the end portions.

2. The needle free injector of claim 1, wherein the shock wave are pulse shock wave.

3. The needle free injector of claim 1, wherein the pressure transmitting unit (30) is an elastic plate.

4. The needle free injector of claim 1, wherein the first electrode (51) is inserted into the insulating unit (53), and
wherein the second electrode (52) is disposed on an outer surface of the insulating unit (53).

5. The needle free injector of claim 1, further comprising:
a driving fluid storage unit (60) to store a driving fluid; and
a driving fluid circulating unit (70) to circulate the driving fluid stored in the driving fluid storage unit (60) and the driving fluid stored in the pressure chamber (10).

6. The needle free injector of claim 1, further comprising:
a medicine storage unit (80) to store the medicine provided in the medicine chamber (20); and
a check valve (90) to prevent the medicine, which is received in the medicine chamber (20), from flowing back to the medicine storage unit (80).

7. The needle free injector of claim 1, further comprising:
a power unit (40) to supply pulsed power to the electrode unit
(50), wherein the power unit (40) includes:
a power supply unit (41);
an electricity storage unit (42) to store voltage and current supplied from the power supply unit (41) in a form of electrical energy; and
a switch (43) to apply the electrical energy, which is stored in the electricity storage unit (42), in a form of pulsed power.

## Patentansprüche

1. Nadelloser Injektor basierend auf Unterwasserentladung unter Verwendung von Stoßwellen, wobei der nadellose Injektor aufweist:
eine Druckkammer (10), in welcher ein Treibfluid aufgenommen ist;
eine mit der Druckkammer (10) kommunizierende Medikamentenkammer (20) zum Aufnehmen von Medikamenten;
eine Druckübertragungseinheit (30), die zwischen der Druckkammer (10) und der Medikamentenkammer (20) angeordnet ist, wobei die Druckübertragungseinheit (30) auf das in der Medikamentenkammer (20) aufgenommene Medikament einen Vortriebsdruck aufbringt, wenn ein Volumen der Druckkammer (10) expandiert wird;
eine Injektionseinheit (21), die auf einer Seite der Medikamentenkammer (20) vorgesehen ist, um das Medikament auszugeben; und
eine Elektrodeneinheit (50) zum Expandieren des Volumens der Druckkammer (10) durch Erzeugen von Blasen in dem in der Druckkammer aufgenommenen Treibfluid und durch Bewirken eines Durchschlags,
**dadurch gekennzeichnet, dass** die Elektrodeneinheit (50) aufweist:
eine erste Elektrode (51) und eine zweite Elektrode (52), um die Stoßwelle zu erzeugen;
wobei die Stoßwelle das Volumen der Druckkammer expandiert, und
eine Isoliereinheit (53), die zwischen der ersten Elektrode (51) und der zweiten Elektrode (52) angeordnet ist,
wobei die erste Elektrode (51), die zweite Elektrode (52) und die Isoliereinheit (53) voneinander verschiedene Längen aufweisen,
wobei ein Endabschnitt der ersten Elektrode (51), ein Endabschnitt der zweiten Elektrode (52), und ein Endabschnitt der Isoliereinheit (53), welche außerhalb der Druckkammer (10) angeordnet sind, zur Bildung gestufter Abschnitte unter den Endabschnitten angeordnet sind.

2. Nadelloser Injektor nach Anspruch 1, bei welchem die Stoßwelle eine Pulsstoßwelle ist.

3. Nadelloser Injektor nach Anspruch 1, bei welchem die Druckübertragungseinheit (30) eine elastische Platte ist.

4. Nadelloser Injektor nach Anspruch 1, bei welchem die erste Elektrode (51) in die Isoliereinheit (52) eingesetzt ist, und
wobei die zweite Elektrode (52) auf einer Außenfläche der Isoliereinheit (53) angeordnet ist.

5. Nadelloser Injektor nach Anspruch 1, ferner mit:
einer Treibfluidaufnahmeeinheit (60) zum Speichern eines Treibfluids; und
einer Treibfluidumwälzeinheit (70) zum Umwälzen des in der Treibfluidaufnahmeeinheit (60) aufgenommenen Treibfluids und des in der Druckkammer (10) aufgenommenen Treibfluids.

6. Nadelloser Injektor nach Anspruch 1, ferner mit:
einer Medikamentenaufnahmeeinheit (80) zum Aufnehmen des in der Medikamentenkammer (20) aufgenommenen Medikaments; und
einem Rückschlagventil (90) zum Verhindern des Zurückfließens des in der Medikamentenkammer (20) aufgenommenen Medikaments in die Medikamentenaufnahmeeinheit (80).

7. Nadelloser Injektor nach Anspruch 1, ferner mit:
einer Energieeinheit (40) zum Liefern von gepulster Energie an die Elektrodeneinheit (50), wobei die Energieeinheit (40) aufweist:
einer Energieversorgungseinheit (41);
eine Elektrizitätsspeichereinheit (42) zum Speichern von Spannung und Strom, welche von der Energieversorgungseinheit (41) geliefert werden, in einer Form von elektrischer Energie; und
einen Schalter (43) zum Anlegen der in der Elektrizitätsspeichereinheit (42) gespeicherten elektrischen Energie in einer Form von gepulster Energie.

## Revendications

1. Injecteur sans aiguille basé sur une décharge sous l'eau à l'aide d'une onde de choc, l'injecteur sans aiguille comprenant :
une chambre de pression (10) dans laquelle est contenu un fluide d'entraînement ;
une chambre de médicament (20) communiquant avec la chambre de pression (10) servant à contenir un médicament ;
une unité de transmission de pression (30) interposée entre la chambre de pression (10) et la chambre de médicament (20), dans lequel l'unité de transmission de pression (30) applique une pression de propulsion au médicament contenu dans la chambre de médicament (20), lorsqu'un volume de la chambre de pression (10) est augmenté ;
une unité d'injection (21) disposée d'un côté de la chambre de médicament (20) servant à décharger le médicament ; et
une unité d'électrodes (50) servant à augmenter le volume de la chambre de pression (10), en générant des bulles dans le fluide d'entraînement contenu dans la chambre de pression et en provoquant un éclatement,
**caractérisé en ce que** l'unité d'électrodes (50) comprend :
une première électrode (51) et une seconde électrode (52) servant à générer l'onde de choc ;
dans lequel l'onde de choc augmente le volume de la chambre de pression
et
une unité isolante (53) interposée entre la première électrode (51) et la seconde électrode (52),
dans lequel la première électrode (51), la seconde électrode (52) et l'unité isolante (53) ont des longueurs mutuellement différentes,
dans lequel une partie d'extrémité de la première électrode (51), une partie d'extrémité de la seconde électrode (52) et une partie d'extrémité de l'unité isolante (53), qui sont disposées à l'extérieur de la chambre de pression (10), sont organisées pour former des parties étagées entre les parties d'extrémité.

2. Injecteur sans aiguille selon la revendication 1, dans lequel l'onde de choc est une onde de choc pulsée.

3. Injecteur sans aiguille selon la revendication 1, dans lequel l'unité de transmission de pression (30) est une plaque élastique.

4. Injecteur sans aiguille selon la revendication 1, dans lequel la première électrode (51) est introduite dans l'unité isolante (53), et
dans lequel la seconde électrode (52) est disposée sur une surface extérieure de l'unité isolante (53).

5. Injecteur sans aiguille selon la revendication 1, comprenant en outre :
une unité de contenance de fluide d'entraînement (60) servant à contenir un fluide d'entraînement ; et
une unité de mise en circulation de fluide d'entraînement (70) servant à mettre en circulation le fluide d'entraînement contenu dans l'unité de contenance de fluide d'entraînement (60) et le fluide d'entraînement contenu dans la chambre de pression (10).

6. Injecteur sans aiguille selon la revendication 1, comprenant en outre :
une unité de contenance de médicament (80) servant à contenir le médicament, disposée dans la chambre de médicament (20) ; et
un clapet antiretour (90) servant à empêcher que le médicament, qui est reçu dans la chambre de médicament (20), ne reflue vers l'unité de contenance de médicament (80).

7. Injecteur sans aiguille selon la revendication 1, comprenant en outre :
une unité de puissance (40) servant à délivrer de la puissance pulsée à l'unité d'électrodes (50), dans lequel l'unité de puissance (40) comprend :
une unité d'alimentation en puissance (41) ;
une unité d'accumulation d'électricité (42) servant à accumuler une tension et un courant fournis par l'unité d'alimentation en puissance (41) sous une forme d'énergie électrique ; et
un commutateur (43) servant à appliquer l'énergie électrique, qui est accumulée dans l'unité d'accumulation d'électricité (42), sous une forme de puissance pulsée.
